# EUROPEAN PATENT SPECIFICATION

(11) **EP 4 608 281 B1**
(45) Date of publication and mention of the grant of the patent: **02.09.2026**
(21) Application number: 23798276.4
(22) Date of filing: 17.10.2023
(51) Int. Cl.: A61B 17/02

(54) **A DEVICE FOR USE IN SURGERY**
VORRICHTUNG ZUR VERWENDUNG IN DER CHIRURGIE
DISPOSITIF DESTINÉ À ÊTRE UTILISÉ EN CHIRURGIE

(30) Priority: 27.10.2022 WO PCT/GB2022/052732
(43) Date of publication of application: 03.09.2025
(73) Proprietor: Crescent Health Solutions Limited, Nottingham Nottinghamshire NG2 7EG (GB)
(72) Inventor: GHOSH, Partha Sarathi, Nottingham, Nottinghamshire NG2 7EG (GB); GHOSH, Srotoswini, Nottingham, Nottinghamshire NG2 7EG (GB)
(74) Representative: Barker Brettell LLP
(86) International application number: PCT/GB2023/052688
(87) International publication number: WO 2024/089391

(56) References cited:
- EP-B1- 0 879 018
- CN-A- 107 468 296
- CN-A- 108 158 659
- US-A1- 2018 296 289
- US-A1- 2019 059 870

## Description

The present disclosure relates to a device for use in surgery, e.g. a surgical implement. The disclosure also relates to a method of using such devices.

Surgery may relate to any surgery carried out on a human or animal body.

During surgery, one or more incisions may be made to a patient's skin in order to access tissue and the like located beneath the skin. Further incisions may be made to further tissue to allow access to deeper tissue.

Laparoscopic surgery is a surgical technique in which narrow tubes (trochars) are inserted into a patient through small incisions. Through these trochars, instruments are inserted. A surgeon uses these instruments to manipulate, cut and sew tissue.

Following a surgical procedure being carried out on tissue beneath the skin, holes and incisions may need to be stitched together to assist healing and prevent ruptures or hernias.

For example, during a surgical procedure carried out on a patient's abdomen, one or more incisions may be made to the skin before one or more trochars or other implements pass through the skin, layer of fat and the rectus sheath in order to reach the abdomen. To finish the surgery, holes or incisions in the rectus sheath should be stitched together.

If a surgeon accidentally inserts a needle too far to stich the rectus sheath without lifting the rectus sheath the bowels may be perforated during the process of stitching the rectus sheath potentially leading to complications such as peritonitis and fistula formation etc.

If a surgeon does not insert the needle far enough and the stitch does not pull together the full thickness of the rectus sheath, the stitch may not hold. This may result in the repair being incomplete with one or more gaps or tears in the stitching and the patient's bowels may herniate through the tears or gaps. These situations could be potentially fatal for a patient or lead to significant morbidity which would require additional surgery to repair.

Having to carry out a post-operative repair is time-consuming, costly and may expose the patient to further risks or complications. For example, a post-operative repair may involve the use of a mesh to reinforce an area of tissue. Even after a successful post-operative repair, the presence of such a mesh may increase the risk and/or complexity of any repeat laparoscopic (or other) surgery that the patient may undergo at a later date. Hence, minimising or reducing the likelihood of a need for post-operative repair may be desirable.

It would be beneficial to mitigate or eliminate one or more of the problems associated with the prior art.

US 2019/059870 A1 discloses a retraction device comprising a shaft, a plurality of arms supported by the shaft, the arms being configured to be moveable between a first orientation in which the device assumes an insertion profile for passage through a surgical incision, and a second orientation in which the device assumes a retraction profile.

CN 107468296A discloses a tractive opening device for abdominal cavity walls. The device comprises a hollow tube channel, the tube channel is sleeved with a mobile end which can slide up and down and an opening device body is arranged at the far end of the tube channel.

CN 108158659A discloses a device for use in surgery according to the preamble of claim 1.

A first aspect provides a device, for use in surgery, according to claim 1 With the arm(s) in the deployed configuration, the arm(s) may be brought into contact, directly or indirectly, with an underside of a structure within a human or animal patient's body and the device may then be used to lift the structure within the human or animal patient's body away from one or more neighbouring or surrounding parts within the human or animal patient's body. Consequently, access to the neighbouring or surrounding parts within the human or animal patient's body may be improved, in order, for example, to carry out a surgical procedure or a part thereof. The desired procedure, which may include stitching to repair an incision, may be carried out with a reduced or minimal risk of inadvertently affecting the structure within the human or animal patient's body that is being lifted by the device. The structure within the human or animal patient's body that is lifted, in use, may vary depending upon the nature of the surgery being undertaken. The structure within the human or animal patient's body that is lifted, in use, may include a layer of tissue such as the rectus sheath, one or more blood vessels, one or more nerves or one or more ureters.

In the retracted configuration, no portion of the one or more arms protrude beyond an outer perimeter of the body. In the retracted configuration, the one or more arms may be entirely contained within the outer perimeter of the body.

The body may have a longitudinal axis. The body may extend along the longitudinal axis from the first end to the second end. In the retracted configuration, the one or more arms may extend no further from the or a longitudinal axis than an outer perimeter of the body.

In this way, in the retracted configuration at least a portion of the device may be inserted into a hole, incision or the like, during surgery, having a diameter approximately the same as an outer diameter of the body near to or at the second end. Similarly, at least a portion of the device may be inserted into a trochar or the like, during surgery.

In the deployed configuration, the one or more arms may extend outwards from the body by any suitable distance.

In this way, where at least a portion of the device has been inserted into a hole, incision or the like having a diameter approximately the same as an outer diameter of the body near to or at the second end, the arms may extend out from the body such that the device cannot pass back through the hole, incision or the like.

The device may comprise an actuation portion disposed near to or at the first end. The actuation portion may be configured to allow or assist a user to operate or actuate the actuator.

The body may comprise an elongate shape. The body may comprise a circular cross-section. The body may comprise a substantially constant diameter between the first end and the second end. The body may comprise a tubular shape. A tubular shape may provide an internal cavity in which the actuator may be at least partially received.

In other examples, the body may comprise an oval, square, rectangular, pentagonal, hexagonal or any other suitable cross-sectional shape.

In some examples, the cross-section may not remain constant between the first end and the second end. For example, the cross-section of the body may vary in size and/or shape at one or more points along the length of the body between the first end and the second end.

The body may comprise a curved, rounded, smoothed or chamfered portion near to or at the second end.

As such, the second end of the body may be less likely to damage a patient's tissue when being inserted through a hole, incision, or the like.

The actuator may comprise any suitable mechanical or electronic means operably connected to the one or more arms such that actuation of the actuator causes at least a portion of the one or more arms to move away from the longitudinal axis.

The actuator may be arranged to be movable within the body. The actuator may comprise a movable portion arranged to be movably received at least partially within the body. The movable portion may be configured to form a sliding connection with an internal portion of the body.

**In** some examples the entire actuator may be movable relative to the body and as such the actuator and the movable portion may comprise the same structure. **In** some examples the actuator comprises a component arranged to remain substantially static relative to the body and also comprises the movable portion.

The movable portion may be operably connected to the one or more arms via any suitable means, such as a mechanical connection, for example.

The movable portion may be operable to move in a longitudinal direction relative to the body. The movable portion may be operable to rotate about the longitudinal axis relative to the body.

The movable portion may comprise a rod, shaft, column or the like.

In some examples, the actuator may comprise a cable or the like.

The moveable portion may be arranged to be manually movable by a user.

The actuator may comprise an electronic or electromechanical actuator. The actuator may comprise a solenoid or the like. The movable portion may comprise a moveable armature of the solenoid.

The actuation portion may comprise a handle, lever or the like.

In some examples, the actuation portion may comprise a button, touch screen, touch sensor, switch or the like.

The actuation portion may be arranged to form a mechanical and/or electrical connection with any suitable portion of the actuator. The actuation portion may be connected to, fixedly connected to, or formed integrally with, the actuator.

The button, touch screen, touch sensor, switch or the like may be electronically connected to an electronic actuator such as a solenoid such that actuation of the button, touch screen, touch sensor, switch or the like may cause operation of the electronic actuator.

The actuation portion may be arranged such that movement of the actuation portion, such as the handle, lever, button or the like, causes movement of the actuator. The handle, lever or the like may comprise a pivot arrangement configured such that two separate portions of the actuation portion may be pressed together by a user. The pivot arrangement may be configured such that pressing together of two separate portions of the actuation portion causes movement of the actuator.

The actuator may be configured such that at least a portion extends out from the body. The portion of the actuator configured to extend out from the body may comprise the actuation portion. The actuation portion may be disposed near to or at the first end of the body.

The actuation portion may be configured such that applying a force on the actuation portion causes movement of the actuator in a direction towards the first end of the body or towards the second end of the body. The actuation portion may comprise a portion suitable for a user to press and/or pull relative to the body. In use, pressing or pulling of the actuation portion may cause movement of the actuator.

The movable arms may have any suitable size or shape.

The device may comprise two or more arms. The device may comprise three or more arms. The device may comprise four or more arms. The device may comprise up to 10 arms or up to 20 arms. The device may comprise 2, 3, 4, 5, 6, 7, 8, or more than 8 arms.

The device may be configured such that the one or more arms may be actuated, in use, all together, or may be actuated in discrete groups, each discrete group comprising at least one of the arms. The device may comprise a plurality of separate actuators, each separate actuator being operable to actuate one of the discrete groups of arms.

Each arm may be substantially similar to any other arm. Any one or more arms may be configured differently to any other of the arms.

One or more arms extend between a connection end and a free end. The connection end of one or more arms may be operably connected to the actuator.

In use, the connection end of one or more arms may remain substantially close to the body. In use, the connection end of one or more arms may remain substantially within the body.

The body comprises one or more apertures disposed near to or at the second end. The one or more apertures may be configured such that at least a portion of one or more arms may extend therethrough. In this way, the one or more apertures may be configured such that, in use, the connection end may be disposed within the body and the free end may extend through an aperture and outside of the body.

The one or more apertures are configured such that at least the free end of one or more arms can move from a region within the body and through the aperture to a region outside of the body, and vice versa. In other examples, any portion of one or more arms may be operable to pass through any aperture, in use.

Each aperture may be configured such that at least one arm may extend therethrough.

Each aperture may be configured such that more than one arm may extend therethrough.

The apertures may be spaced apart from a terminal point of the second end of the body in the longitudinal direction. The apertures may be any suitable distance along the longitudinal axis from the terminal point of the second end of the body. In this way, the free ends of the apertures may extend through the body at a distance away from the terminal point of the second end. In use, the terminal point may act to bias any tissue or other material away from the apertures and as such may prevent the free ends of the arms from contacting any tissue when moving into the deployed configuration.

In the retracted configuration, the arms may be disposed substantially within the body. In the deployed configuration, at least a portion of one or more arms may be disposed further from the longitudinal axis than in the retracted position. In examples, in the deployed configuration the free end of the arms may be disposed further from the longitudinal axis than in the retracted configuration.

Each arm may be spaced apart from any other arm. Each arm may be equally spaced apart from any other arm. Each arm may be unequally spaced apart from any other arm.

The arms may each connect to the actuator via a connecting means configured to allow relative movement between the actuator and the arms, such as a pivot or a hinge, for example. In other examples, the arms may each connect to the actuator via any suitable means.

The actuator and the arms may be configured such that movement of the actuator towards the first end or the second end of the body, or rotational movement of the actuator relative to the body, causes the free end of the arms to move away from the longitudinal axis.

In the deployed configuration, the arms may be orientated substantially perpendicularly to the longitudinal axis of the body. In the deployed configuration, the free end of the arms may be disposed furthest from the longitudinal axis of the body.

In the deployed configuration, the free end of the arms may define points on the circumference of a nominal circle having a greater diameter than the diameter of the body.

In the deployed configuration the arms may be disposed substantially within a plane that is orientated perpendicular to the longitudinal axis. In the deployed configuration, the plane within which the arms are disposed may be spaced apart from the terminal point of the second end of the body. The plane in which the arms sit may be spaced apart by any suitable distance along the longitudinal axis from the terminal point of the second end of the body.

In the retracted configuration, the free end of each arm may be disposed near to or at the one or more apertures. In the retracted configuration, the arms may be orientated substantially parallel to the longitudinal axis of the body. In the retracted position, the arms may be disposed between the actuator and an inner surface of the body. The actuator may comprise a groove, recess, cut out or the like configured to at least partially receive an arm when in the retracted configuration. In the retracted configuration, the arms may be disposed at least partially, or entirely, within the body.

In use, the actuator may be moved in the direction of the second end. The free end of the arms may each interact with an aperture such that the free end of the arms is guided through the apertures in a direction away from the longitudinal axis of the body. As the actuator is moved further towards the second end of the body, the free end of the arms may move further from the longitudinal axis of the body. As the actuator is moved further towards the second end of the body, a greater portion of the arms may move through the apertures.

In examples, the actuator may be configured to move towards the second end of the body until the arms are in the deployed configuration. The actuator may be configured to move back towards the first end of the body and thereby cause the arms to retract back into the retracted configuration.

In examples, the arms may be biased away from the longitudinal axis of the body by any suitable biasing means. The biasing means may comprise a spring, or the like. In this way, the actuator may be configured to move the arms in the longitudinal direction towards the apertures and the biasing means may be configured to move the arms from the retracted to the deployed configuration. The actuator may be configured to then move the arms in the opposing direction and the arm may interact with a portion of the apertures to move the arms from the deployed to the retracted configuration.

In other examples, the actuator may be configured to move the arms from the retracted configuration to the deployed configuration by any other suitable means.

The actuator may be configured such that it can move towards the second end of the body until it reaches a pre-determined limit. Movement of the actuator towards the second end of the body may be limited by any suitable means, such as a stopper, for example.

The actuator may be configured to be movable towards the second end of the body until the arms are in the deployed configuration. The actuator may be configured to be movable back towards the first end of the body and thereby cause the arms to retract back into the retracted configuration.

In examples, the actuator may be configured to rotate relative to the body. The actuator may be configured such that rotation in a first direction causes the arms to move from a retracted configuration into a deployed configuration. The actuator may be configured such that rotation in a second direction causes the arms to move from a deployed configuration into a retracted configuration. The actuator may be connected to the arms via a cam arrangement, or the like.

The device may comprise a temporary securing means operable to temporarily prevent the actuator from moving relative to the body. The temporary securing means may be operable to temporarily prevent the actuator from moving relative to the body when the arms are in the deployed configuration and/or in the retracted configuration.

The temporary securing means may comprise a clamp, for example. The temporary securing means may comprise cooperating features disposed on the body and the actuator. For example, the body may comprise a notch, groove, recess or the like and the actuator may comprise a protrusion configured to be at least partially received into the notch, groove, recess or the like, or vice versa. The notch, groove, recess or the like and the protrusion may be located on each of the body and actuator such that the actuator is temporarily prevented from moving relative to the body when the arms are in the deployed configuration.

The temporary securing means may comprise a "pen click" mechanism or the like. The actuating portion and the actuator may be operably connected by one or more cooperating surfaces, such as cam surfaces, configured to provide a "pen click" mechanism, for example.

The device may comprise one or more means for increasing a user's grip and/or control of the device. The means for increasing a user's grip and/or control of the device may be described as a gripping portion. The gripping portion may comprise one or more protrusions disposed at any suitable location, such as, near to or at the first end, for example. The gripping means may be connected to or formed integrally with the body. The one or more protrusions may be configured such that a user may bias the body in an opposing direction to a force applied to the actuation portion. In this way, a user may use one or more fingers to apply a force to the body in a first direction and use a thumb to apply a force to the actuation portion in an opposing direction. In this way, the device may be configured such that a user can use one hand to hold the body of the device in a substantially fixed position and simultaneously operate the actuator to move the arms from the retracted configuration to the deployed configuration.

The device may comprise at least one light source. The light source may comprise one or more LEDs, or the like. The light source may be disposed near to or at the second end of the body. The light source may be attached to the second end of the body. The light source may be arranged to emit light from the device in one or more directions, which may include a direction forward of the device.

The provision of a light source may be beneficial, since it may provide illumination to help a user, e.g. a surgeon, to use the device to lift a structure within a human or animal patient's body. As a result of the device comprising a light source, there may be no need for a user, e.g. a surgeon, to operate and manipulate simultaneously a separate device with a light source. As a result of the device comprising a light source, the device may be better adapted for use without any trochars in place.

The light source may be powered by any suitable power source, such as a battery, for example. The battery may be disposed within the body.

The device may be configured to be used with an external light source. The external light source may comprise any suitable light source external to the body. For instance, the external light source may comprise a light source disposed in or on a laparoscopic stack or at another location within a site of use, e.g. within an operating theatre. In an implementation, the external light source may be provided in a housing that can be attached to the device and, optionally, detached from the device.

One or more light guides may be configured to direct light from the light source to a desired portion of the device, to provide illumination during use of the device. In an implementation, one or more light guides may be configured to direct light from the external light source into the body and out of the body at a desired location, e.g. out of the second end of the body.

One or more of the light guides may be flexible at least in part.

One or more of the light guides may comprise a light cable.

One or more of the light guides may include a fibre optic element.

One or more of the light guides may comprise a light guide element configured to reflect or refract a beam of light originating from the light source.

One or more of the light guides may be detachable or removable from the body and/or may be reusable at least in part and/or sterilisable at least in part.

The device may comprise a button, switch or the like operably connected to the light source. The button, switch or the like may be configured to control the operation of the light source. In use, a user may press the button or switch in order to turn the light source on and/or off. The button or switch operably connected to the light source may be disposed near to or at the first end of the body.

The device may comprise a fluid channel suitable for conveying a fluid therethrough. The fluid channel may comprise a conduit, passageway or the like. The fluid channel may comprise a fluid inlet, which may be disposed near to or at the first end of the body. The fluid channel may comprise a fluid outlet, which may be disposed near to the second end of the body. In an example, the one or more apertures disposed near to or at the second end of the body may provide the fluid outlet. As such, the fluid channel may, for example, extend along a substantial portion of the length of the body.

The fluid inlet may comprise any suitable valve or the like operable to control the flow of a fluid therethrough. The fluid inlet may be adapted for connection to a fluid supply. Flow of the fluid from the fluid supply may be controlled by a fluid supply valve. In this way, in use, a flow of fluid may be conveyed through the fluid channel, in use, to inflate an internal region of a patient during surgery.

Typically, the fluid may comprise a gas such as carbon dioxide (CO₂) or air. Carbon dioxide is typically used in laparoscopic surgery.

A stream of fluid, e.g. carbon dioxide, may be introduced, e.g. pumped, into the human or animal patient's body to inflate a region local to the second end of the device. Accordingly, a gap may be created and/or maintained to facilitate safer movement of the arms into the deployed position and the subsequent engagement between the arms and an underside of the structure being lifted.

The device may include a sealing element configured to provide a seal, in use, around at least a portion of a perimeter of the body. The seal may act to reduce or substantially prevent fluid escaping, in use, from a patient's body around the outside of the device.

The sealing element may extend around a perimeter, e.g. a circumference, of the body.

The sealing element may be deployable, in use. The sealing element may be inflatable at least in part.

The sealing element may be disposed between the one or more movable arms and the first end of the body.

The device may comprise any suitable materials. The body and/or arms may comprise a metallic material, for example an alloy such as stainless steel.

The body may have an outer diameter of approximately 10mm. The body may have an outer diameter of between approximately 8mm and 10mm. The body may have an outer diameter of at least 4mm, 6mm, 8mm, 10mm, 12mm, 14mm or at least 16mm. The body may have an outer diameter of up to 8mm, 10mm, 12mm, 14mm, 16mm, 18mm or up to 20mm.

The arms may extend any suitable distance from the body. The free end of the arms, or the points of the arms furthest from the body, may define points on the circumference of a nominal circle having any suitable diameter. The longitudinal axis of the body may be the centre of the nominal circle.

In the deployed configuration the arms may define points on the circumference of a nominal circle having a diameter of approximately 28mm. In the deployed configuration the arms may define points on the circumference of a nominal circle having a diameter of between approximately 24mm and 32mm. In the deployed configuration the arms may define points on the circumference of a nominal circle having a diameter of at least 12mm, 16mm, 20mm, 24mm, 28mm, 32mm, 36mm, or 40mm. In the deployed configuration the arms may define points on the circumference of a nominal circle having a diameter of up to 24mm, 28mm, 32mm, 36mm, 40mm, 44mm, or 48mm.

The body may have a length of at least 100mm, 200mm or 300mm. Alternatively or in addition, the body may have a length of less than 150mm, 250mm or 300mm. For example, the body may have a length of approximately 178mm.

It will be understood that the device may have a diameter according to its intended use. The dimensions of the device could be scaled down for use in surgery on small animals relative to surgery on humans, for example. The dimensions of the device could be scaled up or down for use in different types of surgery on humans requiring different sized incisions or holes, or for use in surgery on children relative to adults, for example.

A device of the first aspect may be used:
a) with the one or more movable arms in the retracted configuration, inserting the second end of the device into a human or animal patient's body;
b) actuating the actuator such that at least one of the movable arms move from the retracted configuration into the deployed configuration;
c) optionally, manoeuvring the device so as to use the arm(s) in the deployed configuration to lift a structure within the human or animal patient's body away from one or more neighbouring or surrounding parts within the human or animal patient's body;
d) actuating the actuator such that arm(s) return to the retracted configuration from the deployed configuration; and
d) with the arms in the retracted position, removing the device from the human or animal patient's body.

In the deployed configuration, the one or more arms may extend outwards from the body by any suitable distance.

The structure within the human or animal patient's body that is lifted away from one or more neighbouring or surrounding parts within the human or animal patient's body may vary depending upon the nature of the surgery being undertaken. The structure within the human or animal patient's body that is lifted away from one or more neighbouring or surrounding parts within the human or animal patient's body may include a layer of tissue such as the rectus sheath, one or more blood vessels, one or more nerves or one or more ureters.

The second end of the device may be inserted through a layer of a patient's tissue such as the patient's rectus sheath.

Where the second end of the device has been inserted through a hole, incision or the like through a layer of a patient's tissue, such as the patient's rectus sheath, in the deployed configuration the one or more arms may extend out from the body such that the device cannot pass back through the hole, incision or the like.

During surgery, in particular during a surgical procedure carried out on a patient's bowels, one or more incisions may be made to the skin of the patient's abdomen before one or more trochars or other implements pass through the skin, layer of fat and the rectus sheath in order to reach the bowels. The rectus sheath is a resilient fibrous compartment that contains both the rectus abdominus muscle and the pyramidalis muscle.

It should be understood that whilst a specific surgery may be described herein in detail, the disclosure may equally apply to other surgeries where incisions, tears or holes in one or more layers of tissue may be stitched or operated on in isolation from adjacent layers. It should also be understood that whilst surgery on a human is described herein, the same may be applied to veterinary surgery on an animal.

In one example, once a procedure has been carried out on the abdomen of a patient, the hole in the rectus sheath will need to be repaired by stitching.

A trochar may remain extending through a hole in the rectus sheath when any other surgical implements have been removed from the trochar. Prior to removal of the trochar from the rectus sheath, the second end of the device may be inserted into the trochar. The device may be moved along the trochar until the second end of the device protrudes out from the trochar on the inner side of the rectus sheath. In this way, the second end of the device is located on the inner side of the rectus sheath, and the first end of the device is located on the external side of the rectus sheath.

In some examples, where the device comprises a light source such as an LED, the light source may be activated by a user prior to or after removal of the trochar. In some examples, the light source may be activated prior to the device being inserted into the trochar.

Next, the user may hold the device substantially stationary and remove the trochar. In this way, the trochar may be removed from the patient and the device remains extending through the rectus sheath.

In some examples, the trochar may be removed first and the second end of the device may be pushed through the hole or incision in the rectus sheath such that the device is extending therethrough.

The user may then activate the actuator using the actuation portion. For example, the user may apply a force to the actuation portion in the direction of the second end of the device whilst holding the body substantially stationary. In this way, the actuator may move towards the second end of the device.

In some examples the actuator may comprise an electronic actuator such as a solenoid. In such examples, the user may activate the actuator by pressing a button or switch which may cause the solenoid to extend an armature. The button or switch may again be pressed in order to activate the solenoid to retract the armature.

Movement of the actuator in this way may cause the arms to move from the retracted configuration to the deployed configuration. The free end of the arms may move from within the body and pass through the one or more apertures. The arms may continue to extend away from the longitudinal axis of the body such that the free end of the arms define a nominal circle having a greater diameter than the diameter of the body of the device.

Once in the deployed configuration, a user may hold the actuator stationary relative to the body by maintaining a biasing force on the actuation portion. In some examples, the temporary securing means may act to hold the actuator stationary relative to the body.

Once the arms are in the deployed configuration, the device may no longer be withdrawn back through the hole in the rectus sheath. A user may apply a force to the body in the direction of the first end of the device. In this way, the arms may be biased towards an underside, e.g. an inner surface, of the rectus sheath. As the arms extend across a greater diameter than the body of the device and the diameter of the hole in the rectus sheath, the body may not pass back through the rectus sheath. Instead, the arms will contact the inner surface of the rectus sheath and a force applied to the body by a user in the direction of the first end of the device may cause the rectus sheath to move in the same direction.

By moving the rectus sheath in this way, the rectus sheath may be partially spaced apart from the bowels. A user may be able to hold the device in one hand and as such may hold a portion of the rectus sheath away from the patient's bowels using one hand. The user may then insert one or more stitches through opposing sides of the hole in the rectus sheath. In this way, by partially spacing the rectus sheath away from the bowels, a user may more easily thread a stitch through the full depth of the rectus sheath with a lower risk of perforating the bowels.

The one or more stitches may be left open. The user may allow the body of the device to move back in the direction of the second end of the body and as such move the stitched portion of the rectus sheath back towards the patient's bowels.

In examples where the device comprises a temporary locking mechanism, the user may overcome the temporary locking mechanism by applying a force to the actuation portion and/or by adjusting the temporary locking mechanism such that the actuator is no longer held stationary relative to the body.

The user may then apply a force to the actuation portion in a direction away from the second end of the body. In this way, the actuator moves away from the second end of the body and the arms move from the deployed configuration to the retracted configuration. In this way, the arms may then be retracted back within the body.

The device may then be removed from the rectus sheath and removed from the patient. The user may then tighten the one or more stitches, pulling the opposing sides of the hole in the rectus sheath together.

The method may comprise introducing, e.g. pumping, a stream of fluid, e.g. gaseous carbon dioxide, into the human or animal patient's body to inflate a region local to the second end of the device.

The method may comprise operating a light source to provide illumination within the human or animal patient's body.

The skilled person will appreciate that except where mutually exclusive, a feature or parameter described in relation to any one of the above aspects may be applied to any other aspect. Furthermore, except where mutually exclusive, any feature or parameter described herein may be applied to any aspect and/or combined with any other feature or parameter described herein.

Example embodiments will now be described with reference to the accompanying drawings, in which:
Figure 1 shows a perspective view of an example device for use in surgery, in a retracted configuration;
Figure 2 shows a perspective view of the device shown in Figure 1, in a deployed configuration;
Figure 3 shows a cross sectional view of the second end 6 of the device shown in Figures 1 and 2;
Figure 4 shows an end view of the device shown in Figures 1 and 2 from the second end;
Figure 5 shows a perspective view of the first end of the device shown in Figures 1 and 2;
Figure 6 shows schematically an example first end of a device;
Figure 7 shows schematically an alternative example first end of a device;
Figure 8 shows schematically another alternative example first end of a device;
Figure 9 shows schematically another alternative example first end of a device;
Figure 10 shows schematically another alternative example first end of a device;
Figure 11 shows schematically another alternative example first end of a device;
Figure 12 shows schematically an alternative example second end of a device;
Figure 13 shows schematically another alternative example second end of a device;
Figure 14 shows a portion of an example actuator and four arms;
Figure 15 shows schematically a top down cross sectional view of an example second end;
Figure 16 shows schematically an example second end in use;
Figure 17 shows schematically an example second end in use;
Figure 18 shows schematically an example second end in use;
Figure 19 shows schematically an example second end in use;
Figure 20 shows an example second end in use;

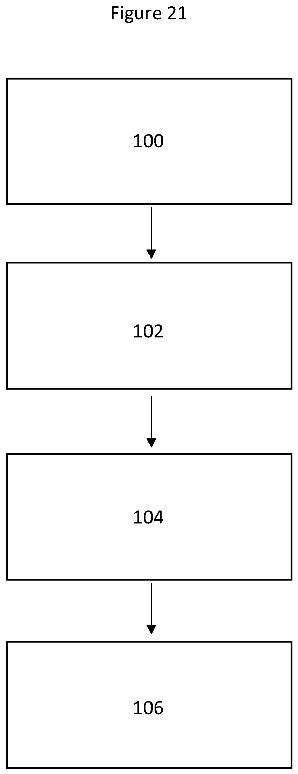

Figure 21 shows an example method of use;
Figure 22 shows an example device for use in surgery; and
Figure 23 shows a second end of another example device for use in surgery.

Where considered appropriate, reference labels have been repeated among the figures to indicate corresponding or analogous elements between alternative examples.

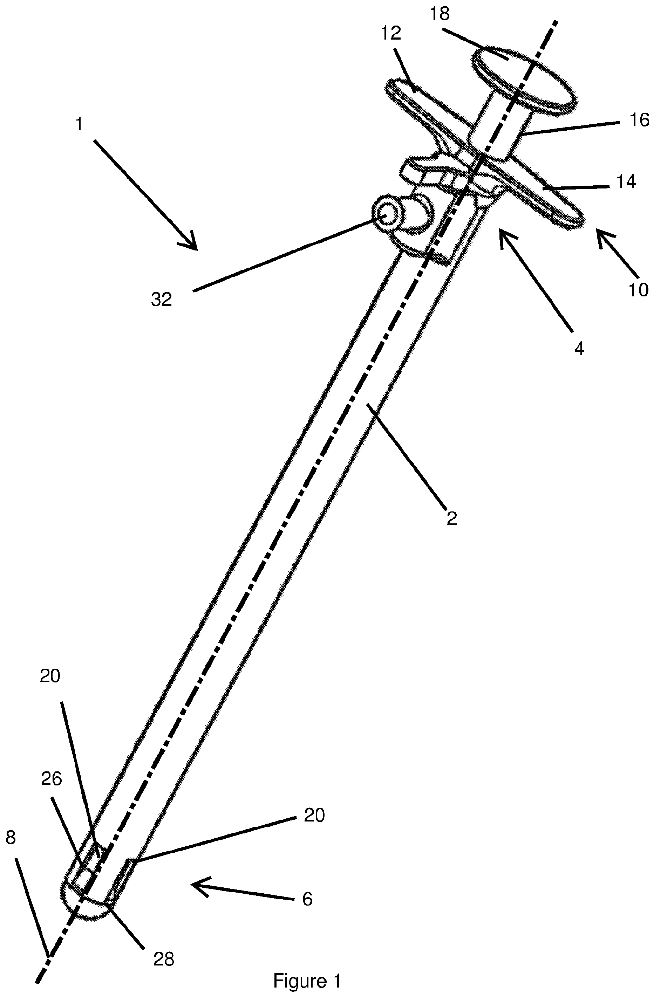

Figure 1 shows an example device 1 for use in surgery, in a retracted configuration. The device 1 comprises a body 2 extending from a first end 4 to a second end 6. The body 2 has an elongate tubular shape with a substantially constant external diameter along its length. The body 2 extends along a longitudinal axis 8.

A gripping portion 10 is disposed at the first end 4 of the device 1. The gripping portion 10 comprises a first support member 12 and a second support member 14 arranged to extend away from the body 2 in opposing directions. The first and second support members 12, 14 are substantially perpendicular to the longitudinal axis 8. In some examples the first and second support members 12, 14 comprise separate portions connected to each other by any suitable means such as an adhesive, for example. In some examples the gripping portion 10 is formed integrally with the body 2. In some examples, the gripping portion 10 is formed separately and then connected to the body 2 by any suitable means. In some examples the device 1 does not comprise a gripping portion 10.

The device 1 comprises an actuator 16 arranged to be at least partially received in the body 2. The actuator 16 is moveably connected to the body 2 and is operable to move within the body 2 along the longitudinal axis 8. In some examples, the actuator 16 is operable to rotate along the longitudinal axis 8 relative to the body 2. In the retracted configuration, the actuator 16 partially extends from the first end 4 of the body 2.

Connected to the actuator 16 is an actuation portion 18. The actuation portion 18 comprises a substantially planar portion of material having a greater diameter than the actuator 16. In this way, the actuation portion 18 cannot be received into the body 2. The actuation portion 18 is configured to allow a user to more easily apply a force to the actuator 16.

In use, a user may use both the actuation portion 18 and the gripping portion 10 with one hand to move the actuator 16 relative to the body 2. For example, a user may position at least one finger on a surface of the gripping portion 10 facing towards the second end 6 of the device 1. A user may then use their thumb to apply a force to the actuation portion 18 in the direction of the second end 6 whilst applying an opposing force to the gripping portion 10. In this way, a user may use one hand to move the actuator 16 towards the second end 6 of the body 2 along the longitudinal axis 8 whilst maintaining the body 2 substantially stationary.

The actuator 16 is operably connected to four arms 20. In other examples, the actuator is operably connected to any suitable number of arms 20. Each arm 20 extends between a connection end 22 and a free end 24 and has a substantially planar body. The connection end 22 of each arm 20 is pivotally connected to the actuator 16.

The arms 20 each comprise a curved portion disposed near to the free end 24. The edges of the arms 20 are rounded such that the arms 20 do not comprise any sharp corners or apexes.

In the example shown in Figure 1, in the retracted configuration the arms 20 are entirely disposed within the circumference of the body 2.

The body 2 comprises four apertures 26 disposed near to the second end 6. Each aperture 26 is associated with an individual arm. Each aperture 26 is configured such that an arm 20 can be deployed through the aperture 26 from an internal region of the body 2 to an external region relative to the body 2, in use.

The arms 20 are operably connected to the actuator 16 such that actuation of the actuator 16 causes the free end 24 of the arms 20 to move through the apertures 26 and away from the longitudinal axis 8.

An LED 28 is disposed at the second end 6. The LED 28 is operable to emit light in one or more directions including a direction forward of the second end 6A button 30 is disposed near to the first end 4 and is connected to the LED 28 such that by pressing the button 30, a user can turn the LED 28 on or off. A battery (not shown) may be disposed within the body 2 and may be arranged to supply power to the LED 28. In some examples the device 1 may not comprise an LED 28. Equally, it will be appreciated that the LED 28 is merely an example of a suitable light source and that, additionally or alternatively, other light sources may be employed at one or more suitable locations, e.g. disposed at or near the second end 6.

A fluid inlet 32 is disposed near to the first end 4. The fluid inlet 32 is configured to allow a flow of fluid, e.g. gaseous carbon dioxide or air, into the body 2. The body 2 is configured such that a flow of fluid may be conveyed from the fluid inlet 32 to the apertures 26, which act as a fluid outlet. For example, in use, a flow of gaseous carbon dioxide may be introduced into a human or animal patient's body to inflate a region local to the second end of the device.

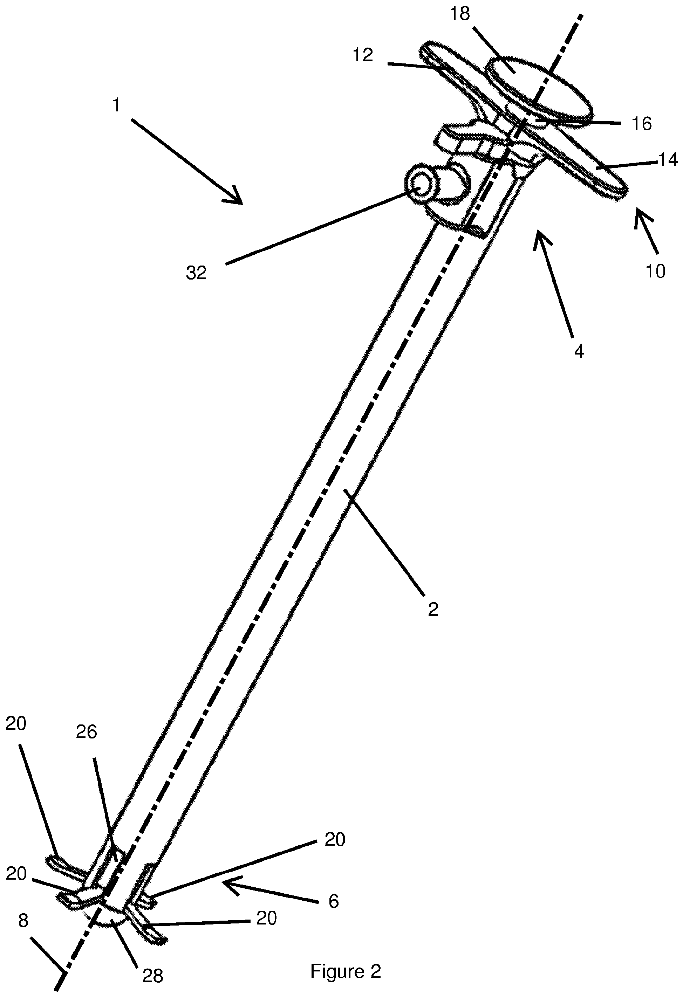

Figure 2 shows the example device 1 shown in Figure 1 but in a deployed configuration.

In use, movement of the actuator 16 towards the second end 6 of the body 2 causes the free end 24 of the arms 20 to contact an edge (not shown) of the associated aperture. As the actuator 16 moves further towards the second end 6 of the body 2, the arms 20 slide along the edge of the associated aperture 26 and as such are angled relative to the longitudinal axis 8 of the body 2. The curved portion of the arms 20 disposed near to the free end 24 assist in directing the free end 24 out of the aperture 26.

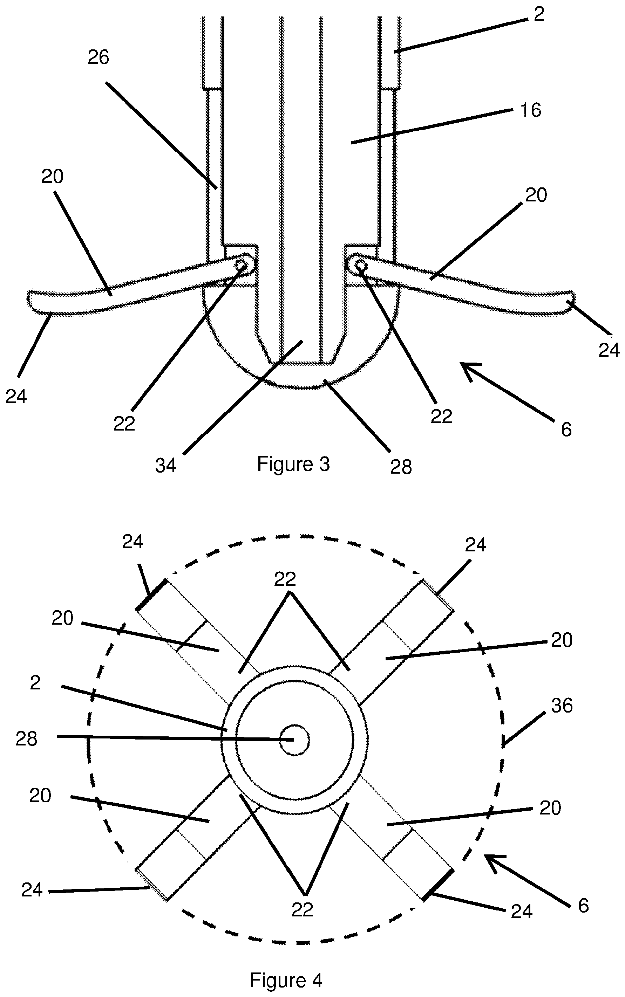

As shown in Figure 3, the connection end 22 of each arm 20 is connected to the actuator 16 within the body 2. The actuator 16 is operable to be moved towards the second end 6 up to a pre-determined limit. At the pre-determined limit, the arms 20 are in the deployed configuration.

The actuator 16 comprises a stopper 34 configured to contact an internal portion of the body 2 near to the second end 6. In use, when the actuator 16 is moved towards the second end 6 of the body 2, the stopper 34 will determine the maximum movement of the actuator 16 in the direction of the second end 6 and as such will determine the pre-determined limit. In some examples the stopper 34 is an integral portion of the actuator 16. In some examples the stopper 34 is connected to the actuator 16 by any suitable means.

In the deployed configuration a user may maintain a force applied to the actuation portion 18 to hold the device 1 in the deployed configuration. In this way, the connection ends 22 of the arms 20 are prevented from moving relative to the body 2. In examples, a temporary securing means (not shown) may be operable to temporarily hold the actuator 16 in a fixed position relative to the body 2. The temporary securing means may comprise cooperating protrusions and notches disposed on the actuator 16 and the body 2 or a latch mechanism or a "pen-click" mechanism, for example.

In the deployed configuration, as shown in Figure 3, the arms 20 are angled relative to the longitudinal axis 8 of the body 2. In examples, in the deployed configuration the arms 20 are angled near to or substantially perpendicular to the longitudinal axis 8.

In the deployed configuration, the connection ends 22 of the arms 20 are prevented from moving relative to the body 2 and the free end 24 of the arms 20 are prevented by the aperture 26 from moving in the direction of the second end 6 of the body 2.

The arms 20 may be retracted back into the retracted configuration by movement of the actuator 16 back towards the first end 4. A user may apply a force to the actuation portion 18 such that the actuator 16 is moved away from the first end 4. In this way, the connection end 22 of the arms 20 will be moved towards the first end 4 of the body 2 until the arms 20 contact an upper edge of the apertures 26.

As the actuator 16 is moved further towards the first end 4 the apertures 26 will engage the arms 20 to bias the arms 20 in towards the longitudinal axis 8 of the body 2. The actuator 16 may be moved towards the first end 4 of the body 2 until the arms 20 are disposed entirely within the body 2 and are back in the retracted configuration.

Figure 4 shows an end view from the second end 6 of the device 1. As shown, the free ends 24 of the arms 20 define points on the circumference of a nominal circle 36. The diameter of the nominal circle 36 defined by the free ends 24 of the arms 20 is greater than the diameter of the body 2.

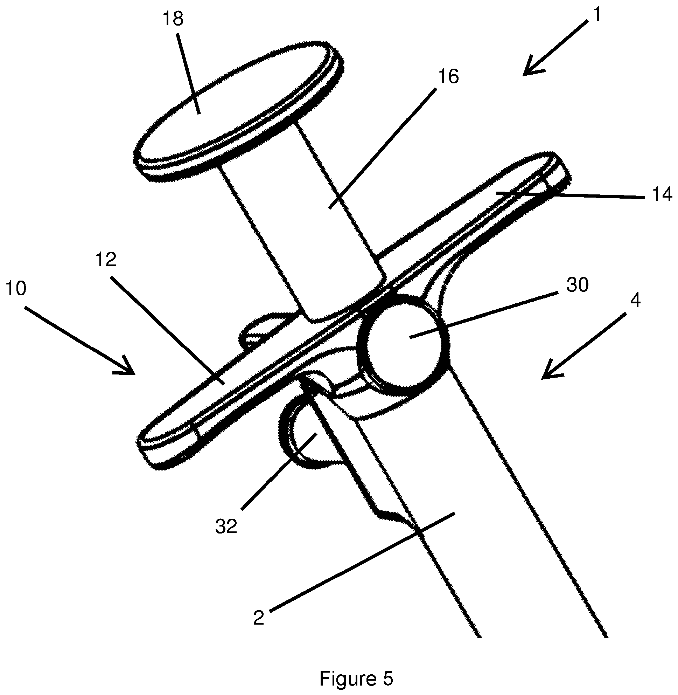

Figure 5 shows the first end 4 of the device 1. The button 30 is shown on the opposing side of the device 1 to the fluid inlet 32. In some examples, the button 30 is replaced by a switch or the like.

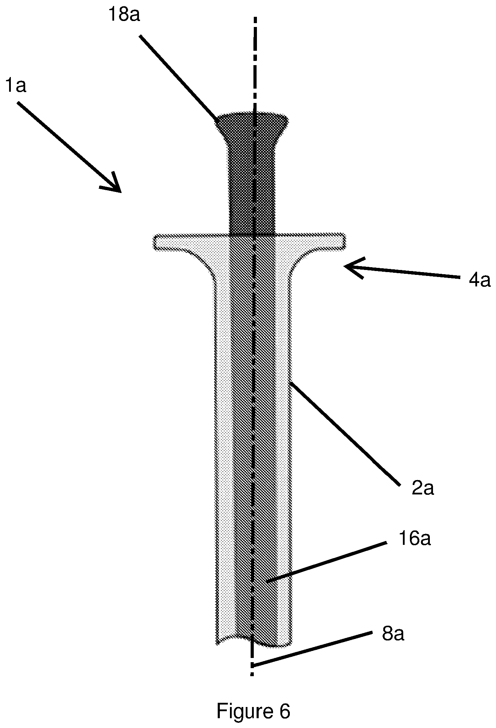

Figures 6 to 11 show schematically example arrangements of manually operated actuation portions 18 and actuators 16. Similar or corresponding features are labelled with similar references. In other examples, the actuator 16 may comprise any suitable electromechanical arrangement such as a solenoid.

Figure 6 shows schematically a cross-sectional view of the first end 4a of an example device 1a. As shown, the actuator 16a is received into the body 2a and extends along the longitudinal axis 8a. The actuation portion 18a comprises a diameter that is greater than the diameter of the actuator 16a. In some examples, the actuator 16a may be operable to rotate relative to the body 2a.

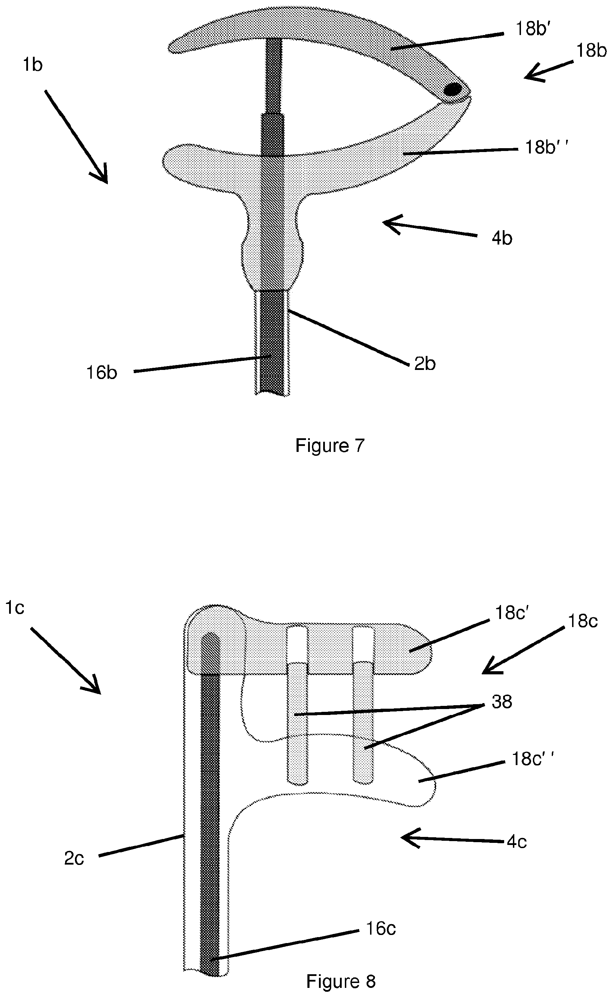

Figure 7 shows schematically a cross-sectional view of the first end 4b of an example device 1b where the actuation portion comprises a handle 18b. The handle 18b comprises a first portion 18b' pivotally connected to a second portion 18b". The first portion 18b' is connected to the actuator 16b such that movement of the first portion 18b' causes movement of the actuator 16b. The second portion 18b" is fixedly connected to the body 2b such that relative movement of the first portion 18b' towards the second portion 18b" causes movement of the actuator 16b relative to the body 2b.

Figure 8 shows schematically a cross-sectional view of the first end 4c of an example device 1c where a handle 18c comprises a first portion 18c' slidably connected to a second portion 18c''. The first portion 18c' is connected to the actuator 16c such that movement of the first portion 18c' causes movement of the actuator 16c. The second portion 18c" is fixedly connected to the body 2c such that relative movement of the first portion towards the second portion 18c" causes movement of the actuator 16c relative to the body 2c. Guide arms 38 are arranged to prevent rotation of the handle 18c relative to each other.

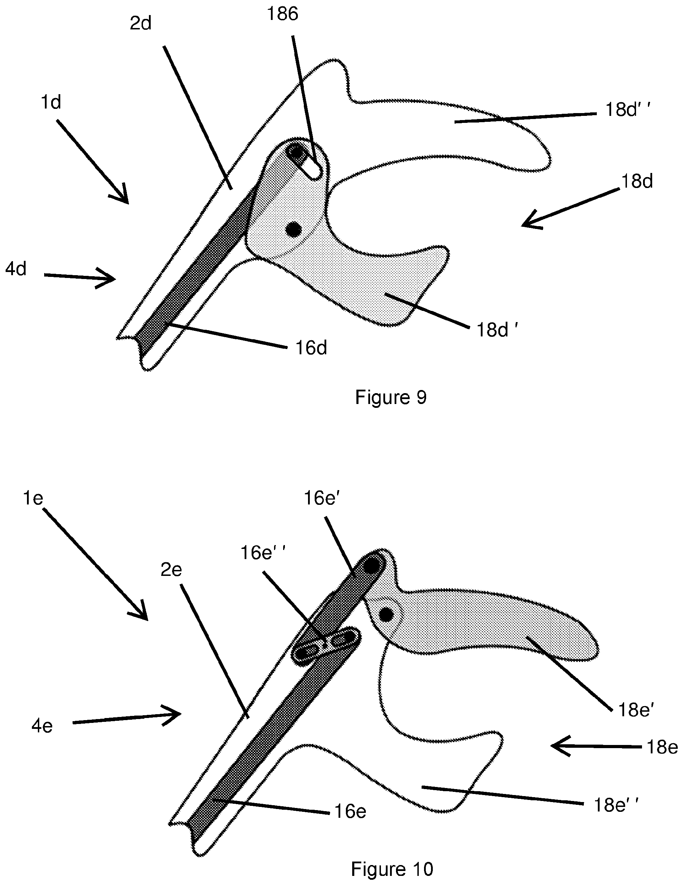

Figure 9 shows schematically a cross-sectional view of the first end 4d of an example device 1d where a handle 18d comprises a first portion 18d' pivotably connected to a second portion 18d". The first portion 18d' is connected to the actuator 16d via a slidable connection portion 186 such that relative movement of the first portion 18d' towards the second portion 18d" causes movement of the actuator 16d. The second portion 18d" is fixedly connected to the body 2d such that movement of the first portion 18d' causes movement of the actuator 16d relative to the body 2d.

Figure 10 shows schematically a cross-sectional view of the first end 4e of an example device 1e where a handle 18e comprises a first portion 18e' pivotably connected to a second portion 18e". The first portion 18e' is connected to the actuator 16e via an intermediate member 16e' such that movement of the first portion 18e' causes movement of the actuator 16e. The intermediate member 16e' is connected to the actuator 16e via a pivoting connector 16e". The second portion 18e" is fixedly connected to the body 2e such that relative movement of the first portion 18e' towards the second portion 18e" causes movement of the actuator 16e relative to the body 2e.

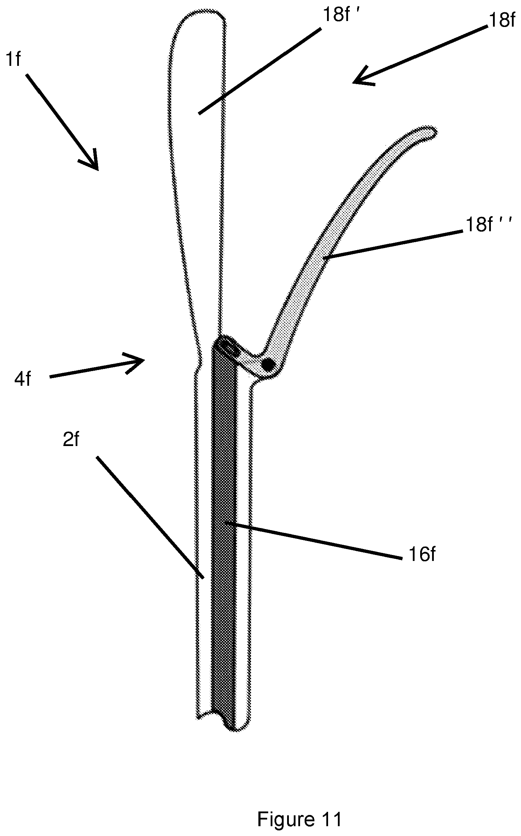

Figure 11 shows schematically a cross-sectional view of the first end 4f of an example device 1f where a handle 18f comprises a first portion 18f ' pivotably connected to a second portion 18f". The first portion 18f ' is connected to the actuator 16f such that relative movement of the first portion 18f 'towards the second portion 18f" causes movement of the actuator 16f. The second portion 18f" is fixedly connected to the body 2f such that movement of the first portion 18f ' causes movement of the actuator 16f relative to the body 2f.

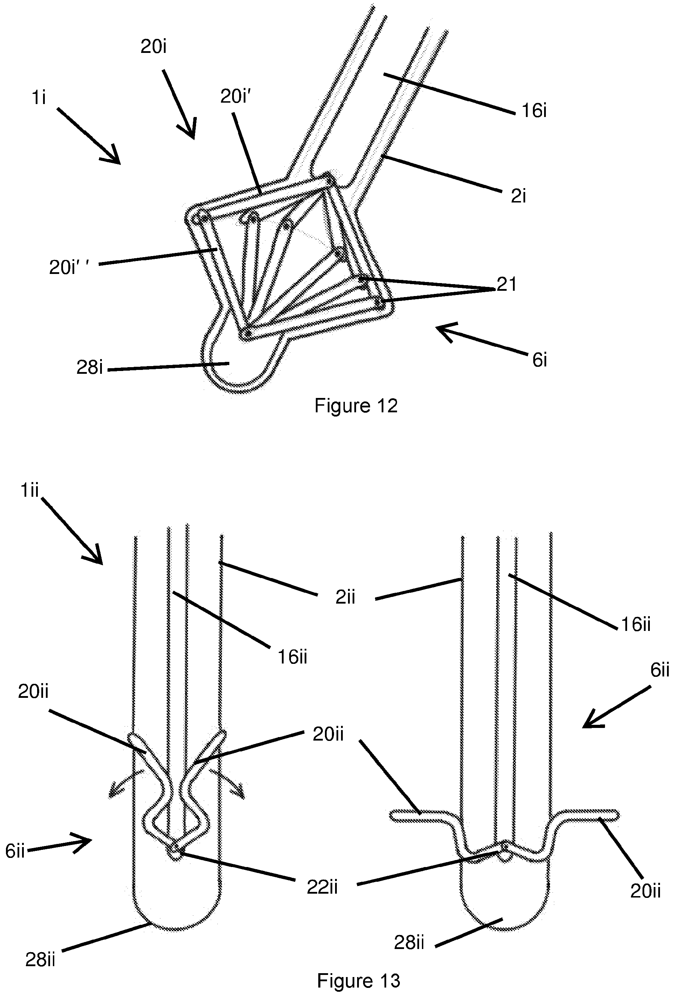

Figure 12 to 16 schematically show example arrangements of one or more arms disposed near to or at the second end 6.

Figure 12 schematically shows schematically an example device 1i having a plurality of movable arms 20i pivotally connected in pairs. Each pair comprises an upper member 20i' movably connected to the actuator 16i, and a lower member 20i" connected within the body 2i near to the second end 6i. Each upper member 20i' is connected to the lower member 20i' ' via a pivot 21. For clarity, not all of the upper arms 20i', lower arms 20i" and pivots 21 are labelled in Figure 12. The pairs are configured such that movement of the actuator 16i towards the second end 6i causes each pair to move such that the pivots 21 connecting each pair move away from the longitudinal axis 8i of the body 2i. A flexible sheath 23 or cover is arranged to surround the arms such that when the pivots 21 extend away from the longitudinal axis 8i of the body 2i, the flexible sheath 23 extends around the arms 20i. The flexible sheath may prevent pinching and entrapment of patient tissue during use of the device.

Figure 13 shows schematically an example device 1ii where the device 1ii may be configured such that movement of the actuator 16ii towards the first end 4ii causes two arms 20ii to move away from the longitudinal axis 8ii of the body 2ii. The two arms 20ii are connected to the actuator 16ii via a pivot 22ii. The body 2ii comprises an aperture 26ii disposed on opposing sides of the body 2ii near to the second end 6ii. The actuator 16ii may be moved in the direction of the first end 4ii by a user. The free ends 24ii of the arms 20ii are arranged to interact with the apertures 26ii such that as the actuator 16ii moves towards the first end 4ii the arms 20ii extend out from the apertures 26ii and away from the longitudinal axis 8ii. When the actuator 16ii is moved back towards the second end 6ii, the arms 20ii will interact with the apertures 26ii to pivot back from the deployed configuration to the retracted configuration.

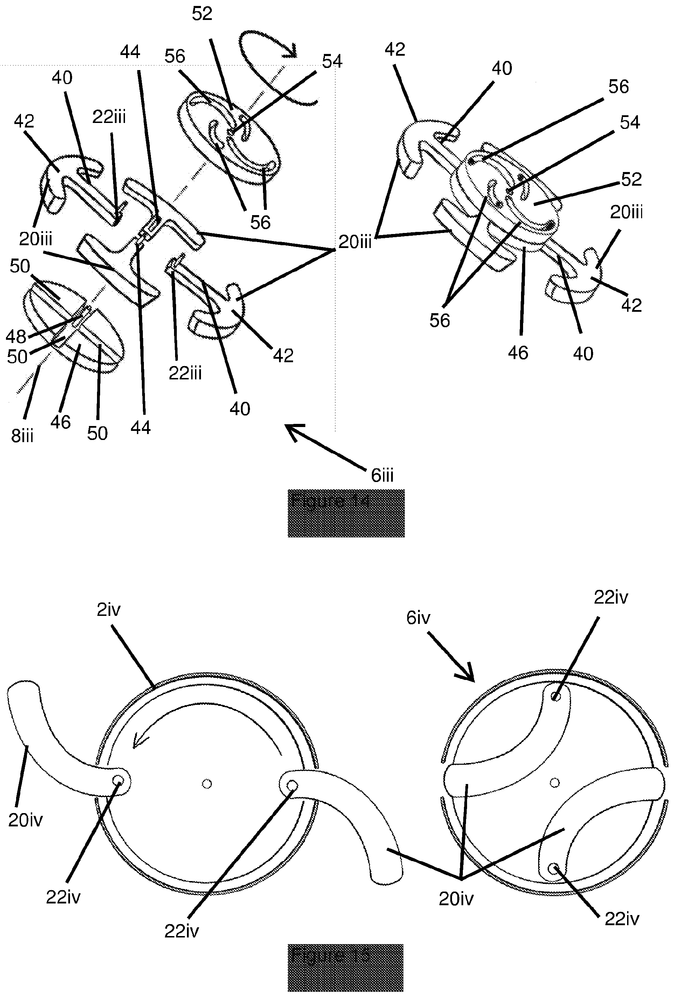

Figure 14 shows an exploded view of an example rotary configuration. Figure 14 shows four arms 20iii spaced apart radially. Each arm 20iii comprises a central arm 40 and a top portion 42. A pin 44 extends from each central arm 40 in a direction parallel to the longitudinal axis 8iii. The central arm 40 extends perpendicularly to the longitudinal axis 8iii of the body and the top portion 42 comprises a curved section. Located towards the second end 6iii of the body is a first lower plate 46 having a central pin 48 and four spaced apart channels 50. The channels 50 are each configured to slidably receive a central arm 40.

Figure 14 also shows an operating plate 52 having a central aperture 54 and four cam slots 56. The operating plate 52 is configured such that the central pin 48 of the lower plate extends through the central aperture 26 and the pin 44 disposed on each of the central arms 40 extends through one of the cam slots 56. The operating plate 52 is configured to be connected to the actuator 16 such that rotation of the actuator 16 causes rotation of the operating plate 52 relative to the body. The lower plate 46 is fixedly connected to the body such that the operating plate 52 is operable to rotate relative to the lower plate 46. As the operating plate 52 rotates in a first direction relative to the lower plate 46, the cam slots 56 are configured to engage the pins of the arms 20iii and move the arms 20iii away from the longitudinal axis 8iii. In this way, the arms 20iii extend away from the body into the deployed configuration. As the operating plate 52 rotates in a second direction relative to the lower plate 46, the cam slots 56 are configured to engage the pins 44 of the arms 20iii and move the arms 20iii back towards the longitudinal axis 8iii and into the retracted configuration.

Figure 15 shows schematically a cross sectional view of another rotary arrangement. The actuator 16 is arranged to rotate relative to the body 2 and is connected to two arms 20iv. The body 2 comprises two apertures 26 and each arm 20iv is arranged such that the free end 24 is disposed near to or at an aperture 26 when in the retracted configuration. When the actuator 16 is rotated in a first direction, the free end 24 of each arm 20 is configured to extend through the aperture 26 and move away from the longitudinal axis 8. When the actuator 16 is rotated in a second direction, the arms 20 are configured to retract back through the apertures 26.

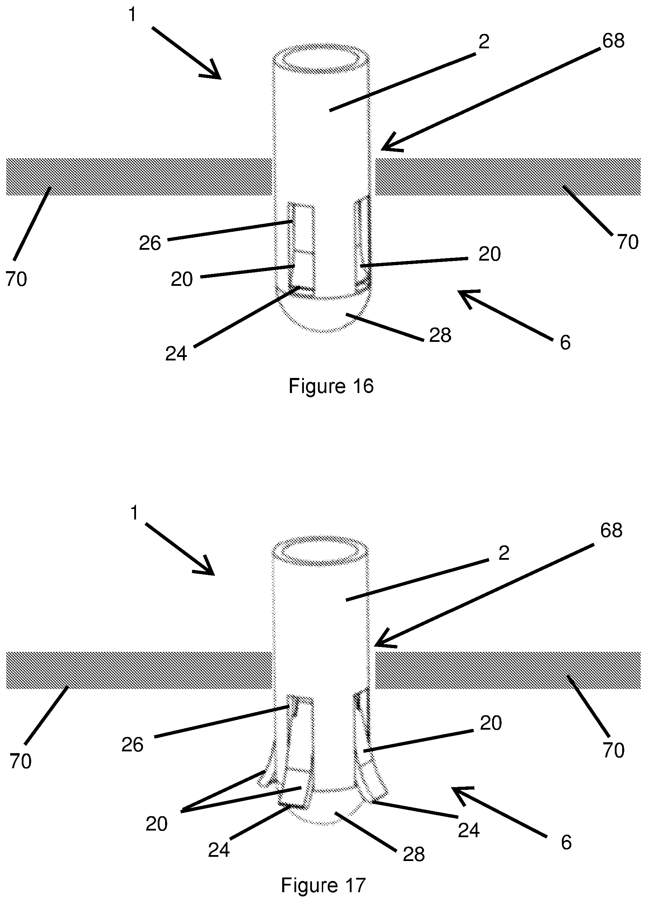

Figures 16 to 19 show the second end 6 of the device 1, in use.

In use, in the retracted configuration the second end 6 of the device 1 may be pushed through a hole 68 or incision in a patient's rectus sheath 70 such that the device 1 is extending therethrough. The device 1 may be inserted through the rectus sheath 70 such that the apertures 26 extend at least partially through to an inner side of the rectus sheath 70. In this way, the arms 20 may extend through the apertures 26 to a region on the inner side of the rectus sheath 70.

The user may then activate the actuator 16 using the actuation portion 18 by applying a force to the actuation portion 18 in the direction of the second end 6 of the device 1 whilst holding the body 2 substantially stationary. In this way, the actuator 16 is moved towards the second end 6 of the device 1.

Movement of the actuator 16 in this way causes the arms 20 to move from the retracted configuration to the deployed configuration. This movement is shown through Figures 16 to 20.

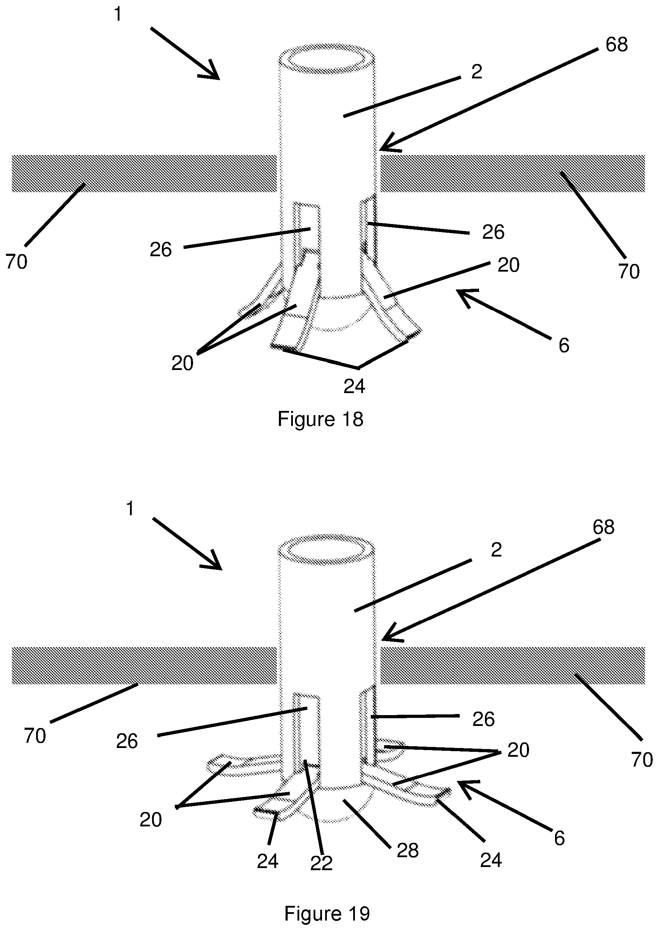

Once the arms 20 are in the deployed configuration, as shown in Figure 19, the device 1 may no longer be withdrawn back through the hole 68 in the rectus sheath 70. A user may apply a force to the body 2 in the direction of the first end 4 of the device 1 such that the arms 20 apply a force to the inner surface of the rectus sheath and cause the rectus sheath 70 to move in the same direction.

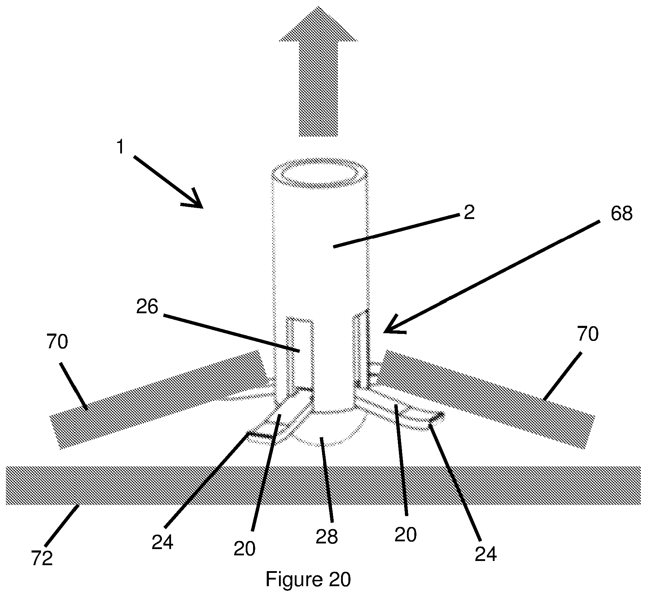

As shown in Figure 20, by moving the rectus sheath 70 in this way, the rectus sheath 70 may be partially spaced apart from a bowels 72. Figure 20 shows how the rectus sheath 70 may be spaced apart from the bowels and in this way provide a gap to assist with inserting a stitch with a lower risk of perforating the bowels 72 or stitching the bowels to the rectus sheath.

The user can then allow the body 2 of the device 1 to move back in the direction of the second end 6 of the body 2 and apply a force to the actuation portion 18 in a direction away from the second end 6 of the body 2. In this way, the actuator 16 moves away from the second end 6 of the body 2 and the arms 20 move from the deployed configuration to the retracted configuration. Once the arms 20 have then been retracted back within the body 2 the device 1 may be removed from the rectus sheath 70 and removed from the patient. The user may then tighten the one or more stitches, pulling the opposing sides of the hole in the rectus sheath 70 together.

Figure 20 is a flowchart of a method of surgery using the device 1. The method comprises the following steps:
a) in a first step 100, inserting the second end 6 of the device through an incision through a layer of tissue of a human or animal patient's body, the incision through the layer of tissue providing access to a surgical site within the patient's body;
b) in a second step 102, actuating the actuator 16 such that at least a portion of the one or more arms move away from the longitudinal axis 8 into the deployed configuration;
c) in a third step 104, actuating the actuator 16 such that at least a portion of the one or more arms move back towards the longitudinal axis 8 into the retracted configuration; and
d) in a fourth step 106, removing the device 1 from the patient's body by moving the second end 6 of the device back through the incision through the layer of tissue.

When the arm(s) is/are in the deployed configuration, the arm(s) may be brought into contact with an inner surface of the layer of tissue, e.g. the rectus sheath, to lift the layer of tissue away from nearby parts of the patient's body. With the layer of tissue lifted away from nearby parts of the patient's body, one or more stitches may be made at or near the surgical site. The risk of stitching the surgical site to the layer of tissue may be reduced or even eliminated through use of a device, e.g. the device 1, according to the present disclosure.

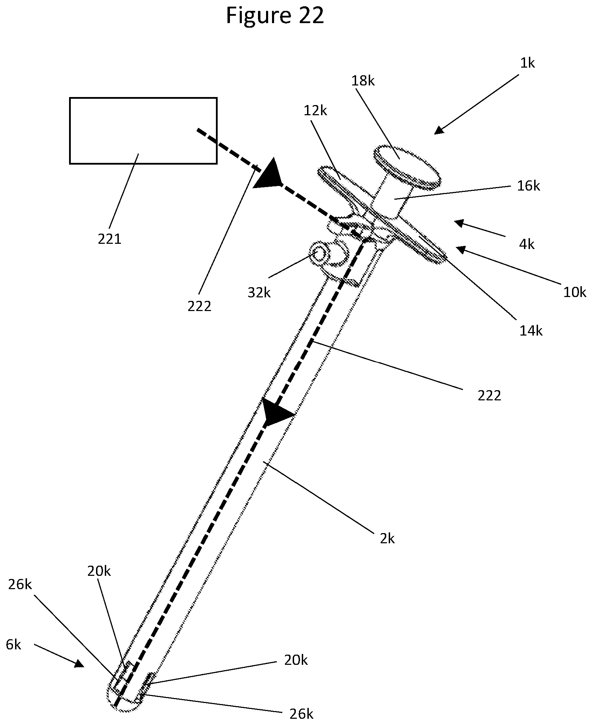

Figure 22 shows an example device 1k for use in surgery, in a retracted configuration. The device 1k operates in substantially the same manner as the device 1 described above. Corresponding features to other implementations described herein are indicated with the same reference number but with a suffix of a "k".

The device 1k comprises a body 2k extending from a first end 4k to a second end 6k. The body 2k has an elongate tubular shape with a substantially constant external diameter along its length.

A gripping portion 10k is disposed at the first end 4k of the device 1k. The gripping portion 10k comprises a first support member 12k and a second support member 14k arranged to extend away from the body 2k in opposing directions. In some implementations, the device 1k may not comprise a gripping portion 10k.

The device 1k comprises an actuator 16k arranged to be at least partially received in the body 2k. The actuator 16k is moveably connected to the body 2k and is operable to move within the body 2k in a longitudinal direction. In the retracted configuration, the actuator 16k partially extends from the first end 4k of the body 2k.

Connected to the actuator 16k is an actuation portion 18k. The actuation portion 18k is configured to allow a user to more easily apply a force to the actuator 16k.

In use, a user may use both the actuation portion 18k and the gripping portion 10k with one hand to move the actuator 16k relative to the body 2k. For example, a user may position at least one finger on a surface of the gripping portion 10k facing towards the second end 6k of the device 1k. A user may then use their thumb to apply a force to the actuation portion 18k in the direction of the second end 6k whilst applying an opposing force to the gripping portion 10k. In this way, a user may use one hand to move the actuator 16k towards the second end 6k of the body 2k whilst maintaining the body 2k substantially stationary.

The actuator 16k is operable connected to four arms 20k. In other implementations, the actuator may be operably connected to any suitable number of arms. Each arm 20k extends between a connection end and a free end and has a substantially planar body. The connection end of each arm 20k is pivotally connected to the actuator 16k.

The arms 20k each comprise a curved portion disposed near to the free end. The edges of the arms 20k are rounded such that the arms 20k do not comprise any sharp corners or apices.

In the example shown in Figure 22, in the retracted configuration the arms 20k are entirely disposed within the circumference of the body 2k.

The body 2k comprises four apertures 26k disposed near to the second end 6k. Each aperture 26k is associated with an individual arm 20k. Each aperture 26k is configured such that an arm 20k can be deployed through the aperture 26k from an internal region of the body 2k to an external region relative to the body 2k, in use.

The arms 20k are operably connected to the actuator 16k such that actuation of the actuator 16k causes the free end of the arms 20k to move through the apertures 26k and away from a longitudinal axis of the body 2k.

A fluid inlet 32k is disposed near to the first end 4k. The fluid inlet 32k is configured to allow a flow of fluid, e.g. gaseous carbon dioxide or air, into the body 2k. The body 2k is configured such that a flow of fluid may be conveyed from the fluid inlet 32k to the apertures 26k, which each act as a fluid outlet. For example, in use, a flow of gaseous carbon dioxide may be introduced into a human or animal patient's body to inflate a region of the human or animal patient's body local to the second end 6k of the device 1k.

The device 1k is configured to be used with an external light source 221. The external light source 221 may comprise any suitable light source external to the body 2k. For instance, the external light source 221 may comprise a light source disposed in a laparoscopic stack or at another location within a site of use, e.g. within an operating theatre. In another implementation, the external light source 221 may be provided in a housing that can be attached and detached from the device 1k.

One or more light guides are configured to direct light from the external light source 221 into the body 2k and out of the second end 6k of the body 2k, as indicated by the dashed lines 222, to provide illumination during use of the device 1k. In implementations, the light guide(s) may be configured to direct light additionally or alternatively out of at least one other portion of the body 2k, i.e. at least one portion of the body 2k other than the second end 6k.

One or more of the light guides may be flexible at least in part.

The light guide(s) may comprise a light cable, e.g. a flexible light cable.

One or more of the light guides may comprise a fibre optic element.

One or more of the light guides may comprise a light guide element configured to reflect or refract a beam of light originating from the external light source 221.

One or more of the light guide(s) may be detachable or removable from the body 2k and/or may be reusable at least in part and/or sterilisable at least in part. In implementations, the body 2k may be intended for a single use in surgery and the light guide(s) may be reusable. By employing an external light source and/or making the light guide(s) detachable or removable from the body 2k, post-surgery waste handling and recyclability of the device 1k may be facilitated.

It will be appreciated that any device disclosed herein may be configured to be used with an external light source, e.g. an external light source similar to the external light source 221.

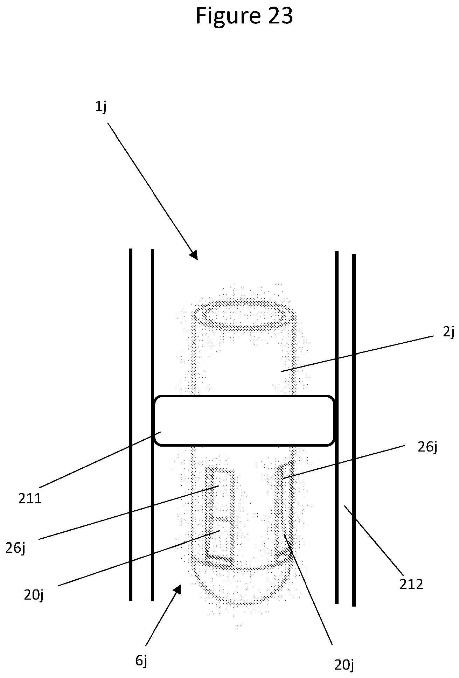

Figure 23 shows a second end 6j of an example device 1j for use in surgery. The device 1j operates in substantially the same manner as the device 1 described above. Corresponding features to other implementations described herein are indicated with the same reference number but with a suffix of a "j".

The device 1j is shown in a retracted configuration. The device 1j is shown within a trochar 212.

The device 1j includes a sealing element 211 configured to form a seal, in use, between the device 1j and an inner wall of the trochar 212. The sealing element 211 is located above the apertures 26j. The sealing element 211 may be deployed, in use, in any suitable manner. For instance, the sealing element 211 may be inflatable such that the seal is formed, in use between the device 1j and the inner wall of the trochar 212 when the sealing element 211 is inflated. The seal formed, in use, between the device 1j and the inner wall of the trochar 212 may act to minimise or prevent any escape of fluid, e.g. gaseous carbon dioxide or air, from the patient's body during surgery via an annulus between the device 1j and the trochar 212.

The same supply of fluid or a different supply of fluid may be employed to inflate the sealing element 211 as to provide the fluid to the fluid inlet.

The sealing element 211 may be configured to minimise or prevent any escape of fluid, e.g. gaseous carbon dioxide or air, from the patient's body when the device 1j is not used with a trochar.

In an implementation, the sealing element 211 may be located on the device 1j at a location that would be below or above the rectus sheath.

It will be appreciated that any device disclosed herein may have a sealing element similar to the sealing element 211.

Throughout the disclosure, the term 'inner' is used to describe a surface or side closest to the core of an object. For example, the inner surface of the rectus sheath is the surface of the rectus sheath closest to and facing the core of the patient.

It will be appreciated that the device according to the present disclosure may be utilised in any type of surgery, in particular laparoscopic surgery. The surgery may be performed on a human or an animal patient.

It will be understood that the invention is not limited to the embodiments described above. Various modifications and improvements can be made without departing from the invention as defined by the claims. Except where mutually exclusive, any of the features may be employed separately or in combination with any other features and the disclosure extends to all combinations and sub-combinations of one or more features disclosed herein.

## Claims

1. A device (1), for use in surgery, comprising:
a body (2) having a first end (4) and a second end (6);
an actuator (16);
one or more movable arms (20) disposed near to or at the second end (6);
wherein the actuator (16) and the one or more movable arms (20) are operably connected such that actuation of the actuator (16) causes the one or more arms (20) to move between a retracted configuration and a deployed configuration, wherein in the deployed configuration at least a portion of the one or more arms (20) is disposed externally from the body (2),
wherein, in the retracted configuration, no portion of the one or more movable arms (20) protrudes beyond an outer perimeter of the body (2), **characterised in that** the one or more arms (20) extend between a connection end (22) and a free end (24), and the body (2) comprises one or more apertures (26) disposed near to or at the second end (6), and the one or more apertures (26) are configured such that at least the free end (24) of one or more arms (20) can move from a region within the body (2) and through the aperture (26) to a region outside of the body (2), and vice versa.

2. A device (1) as claimed in claim 1, wherein the actuator (16) is arranged to be movable within the body (2).

3. A device (1) as claimed in claim 1 or claim 2, wherein the actuator (16) comprises a movable portion arranged to be movably received at least partially within the body (2).

4. A device (1) as claimed in claim 3, wherein:
the movable portion is operably connected to the one or more arms (20); and/or
wherein the movable portion is operable to move in the longitudinal direction relative to the body (2); and/or
wherein the movable portion is operable to rotate about the longitudinal axis relative to the body (2); and/or
wherein the moveable portion is arranged to be manually movable by a user or wherein the actuator (16) comprises a mechanical, electronic or electromechanical actuator, optionally wherein the actuator (16) comprises a solenoid, and further optionally wherein the movable portion comprises a moveable armature of the solenoid.

5. A device (1) as claimed in any preceding claim comprising an actuation portion (18) disposed near or at the first end (4), the actuation portion (18) being configured to allow or assist a user to operate or actuate the actuator (16), optionally wherein the actuation portion (18) comprises a handle, lever, button, touch screen, touch sensor, or switch.

6. A device (1) as claimed in claim 5, wherein the actuation portion (18) is arranged such that movement of the actuation portion (18) causes movement of the actuator (16).

7. A device (1) as claimed in any preceding claim, wherein each of the one or more arms (20) comprise an upper member (20i') and a lower member (20i") pivotably connected to the upper member (20i'), wherein one of; the upper member (20i'), or the lower member (20i"), is directly coupled to the actuator (16) and the other of; the upper member (20i'), or the lower member (20i"), is directly coupled to the body (2), optionally wherein either or both of the lower member (20i") or upper member (20i') is pivotably coupled to the body (2) and to the actuator (16), optionally wherein the one or more apertures (26) are configured such that at least a portion of one or more arms (20) extend therethrough.

8. A device (1) as claimed in any preceding claim, wherein the connection end (22) of one or more arms (20) is operably connected to the actuator (16).

9. A device (1) as claimed in any one of the preceding claims, wherein the apertures (26) are spaced apart from the terminal point of the second end (6) of the body (2) in the longitudinal direction.

10. A device (1) as claimed in any one of the preceding claims, wherein in the deployed configuration, at least a portion of the arm(s) (20) is/are orientated substantially perpendicularly to a longitudinal axis of the body (20).

11. A device (1) as claimed in claim 3 or any one of claims 4 to 11 when dependent on claim 3 comprising a temporary securing means operable to temporarily prevent the actuator (16) from moving relative to the body (2).

12. A device (1) as claimed in any preceding claim comprising at least one light source, optionally wherein the light source comprises one or more LEDs (28) and/or optionally wherein the light source is disposed near to or at the second end (6) of the body (2).

13. A device (1) as claimed in any preceding claim, wherein the device (1) is configured to be used with an external light source (221).

14. A device (1) as claimed in claim 12 or claim 13 comprising one or more light guides configured to direct light from the light source to a desired portion of the device (1).

15. A device (1) as claimed in any preceding claim:
comprising a fluid channel suitable for conveying a stream of fluid therethrough, optionally wherein the fluid channel comprises a fluid inlet (32) disposed near to or at the first end (4) of the body (2) and a fluid outlet disposed near to or at the second end (6) of the body (2); and/or
the device (1) including a sealing element (211) configured to provide a seal, in use, around at least a portion of a perimeter of the body (2).

## Patentansprüche

1. Vorrichtung (1) zur Verwendung in der Chirurgie, umfassend:
einen Körper (2), der ein erstes Ende (4) und ein zweites Ende (6) aufweist;
einen Betätiger (16);
einen oder mehrere bewegbare Arme (20), die nahe oder an dem zweiten Ende (6) vorgesehen sind;
wobei der Betätiger (16) und der eine oder die mehreren bewegbaren Arme (20) derart betreibbar verbunden sind, dass eine Betätigung des Betätigers (16) den einen oder die mehreren Arme (20) dazu veranlasst, sich zwischen einer eingefahrenen Konfiguration und einer ausgefahrenen Konfiguration zu bewegen, wobei in der ausgefahrenen Konfiguration mindestens ein Abschnitt des einen oder der mehreren Arme (20) extern zu dem Körper (2) vorgesehen ist,
wobei, in der eingefahrenen Konfiguration, kein Abschnitt des einen oder der mehreren bewegbaren Arme (20) über einen Außenumfang des Körpers (2) vorsteht, **dadurch gekennzeichnet, dass**
sich der eine oder die mehreren Arme (20) zwischen einem Verbindungsende (22) und einem freien Ende (24) erstrecken und der Körper (2) eine oder mehrere Öffnungen (26) umfasst, die nahe oder an dem zweiten Ende (6) vorgesehen sind, und die eine oder die mehreren Öffnungen (26) derart konfiguriert sind, dass sich mindestens das freie Ende (24) eines oder mehrerer Arme (20) aus einer Region innerhalb des Körpers (2) und durch die Öffnung (26) hindurch zu einer Region außerhalb des Körpers (2) und umgekehrt bewegen kann.

2. Vorrichtung (1) nach Anspruch 1, wobei der Betätiger (16) dazu angeordnet ist, innerhalb des Körpers (2) bewegbar zu sein.

3. Vorrichtung (1) nach Anspruch 1 oder Anspruch 2, wobei der Betätiger (16) einen bewegbaren Abschnitt umfasst, der dazu angeordnet ist, mindestens teilweise innerhalb des Körpers (2) bewegbar aufgenommen zu werden.

4. Vorrichtung (1) nach Anspruch 3, wobei:
der bewegbare Abschnitt betreibbar mit dem einen oder den mehreren Armen (20) verbunden ist; und/oder
wobei der bewegbare Abschnitt dazu betreibbar ist, sich in der Längsrichtung in Bezug auf den Körper (2) zu bewegen; und/oder
wobei der bewegbare Abschnitt dazu betreibbar ist, sich um die Längsachse in Bezug auf den Körper (2) zu drehen; und/oder
wobei der bewegbare Abschnitt dazu angeordnet ist, durch einen Benutzer manuell bewegbar zu sein, oder wobei der Betätiger (16) einen mechanischen, elektronischen oder elektromechanischen Betätiger umfasst, wobei der Betätiger (16) gegebenenfalls einen Magneten umfasst und wobei der bewegbare Abschnitt ferner gegebenenfalls eine bewegbare Armatur des Magneten umfasst.

5. Vorrichtung (1) nach einem der vorhergehenden Ansprüche, umfassend einen Betätigungsabschnitt (18), der nahe oder an dem ersten Ende (4) vorgesehen ist, wobei der Betätigungsabschnitt (18) dazu konfiguriert ist, es einem Benutzer zu ermöglichen oder ihn dabei zu unterstützen, den Betätiger (16) zu betreiben oder zu betätigen, wobei der Betätigungsabschnitt (18) gegebenenfalls einen Griff, einen Hebel, eine Taste, einen Berührungsbildschirm, einen Berührungssensor oder einen Schalter umfasst.

6. Vorrichtung (1) nach Anspruch 5, wobei der Betätigungsabschnitt (18) derart angeordnet ist, dass eine Bewegung des Betätigungsabschnitts (18) eine Bewegung des Betätigers (16) veranlasst.

7. Vorrichtung (1) nach einem der vorhergehenden Ansprüche, wobei jeder des einen oder der mehreren Arme (20) ein oberes Element (20i') und ein unteres Element (20i'') umfasst, das schwenkbar mit dem oberen Element (20i') verbunden ist, wobei eines des; oberen Elements (20i') oder des unteren Elements (20i") unmittelbar an den Betätiger (16) gekoppelt ist und das andere des; oberen Elements (20i') oder des unteren Elements (20i") unmittelbar an den Körper (2) gekoppelt ist, wobei ein beliebiges oder beide des unteren Elements (20i") oder des oberen Elements (20i') gegebenenfalls schwenkbar an den Körper (2) und an den Betätiger (16) gekoppelt sind, wobei die eine oder die mehreren Öffnungen (26) gegebenenfalls derart konfiguriert sind, dass sich mindestens ein Abschnitt eines oder mehrerer Arme (20) dahindurch erstreckt.

8. Vorrichtung (1) nach einem der vorhergehenden Ansprüche, wobei das Verbindungsende (22) eines oder mehrerer Arme (20) betreibbar mit dem Betätiger (16) verbunden ist.

9. Vorrichtung (1) nach einem der vorhergehenden Ansprüche, wobei die Öffnungen (26) in der Längsrichtung von dem Beendigungspunkt des zweiten Endes (6) des Körpers (2) beabstandet sind.

10. Vorrichtung (1) nach einem der vorhergehenden Ansprüche, wobei, in der ausgefahrenen Konfiguration, mindestens ein Abschnitt des Armes/der Arme (20) im Wesentlichen senkrecht zu einer Längsachse des Körpers (20) ausgerichtet ist.

11. Vorrichtung (1) nach Anspruch 3 oder einem der Ansprüche 4 bis 11, wenn von Anspruch 3 abhängig, umfassend ein vorübergehendes Sicherungsmittel, das dazu betreibbar ist, den Betätiger (16) vorübergehend daran zu hindern, sich in Bezug auf den Körper (2) zu bewegen.

12. Vorrichtung (1) nach einem der vorhergehenden Ansprüche, umfassend mindestens eine Lichtquelle, wobei die Lichtquelle gegebenenfalls eine oder mehrere LEDs (28) umfasst und/oder wobei die Lichtquelle gegebenenfalls nahe oder an dem zweiten Ende (6) des Körpers (2) vorgesehen ist.

13. Vorrichtung (1) nach einem der vorhergehenden Ansprüche, wobei die Vorrichtung (1) dazu konfiguriert ist, mit einer externen Lichtquelle (221) verwendet zu werden.

14. Vorrichtung (1) nach Anspruch 12 oder Anspruch 13, umfassend einen oder mehrere Lichtleiter, die dazu konfiguriert sind, Licht von der Lichtquelle zu einem erwünschten Abschnitt der Vorrichtung (1) zu richten.

15. Vorrichtung (1) nach einem der vorhergehenden Ansprüche:
umfassend einen Fluidkanal, der dazu geeignet ist, einen Fluidstrom dahindurch zu fördern, wobei der Fluidkanal gegebenenfalls einen Fluideinlass (32), der nahe oder an dem ersten Ende (4) des Körpers (2) vorgesehen ist, und einen Fluidauslass, der nahe oder an dem zweiten Ende (6) des Körpers (2) vorgesehen ist, umfasst; und/oder
wobei die Vorrichtung (1) ein Abdichtungsteil (211) beinhaltet, das dazu konfiguriert ist, bei Verwendung eine Abdichtung um mindestens einen Abschnitt eines Umfangs des Körpers (2) bereitzustellen.

## Revendications

1. Dispositif (1), destiné à être utilisé en chirurgie, comprenant :
un corps (2) ayant une première extrémité (4) et une seconde extrémité (6) ;
un actionneur (16) ;
un ou plusieurs éléments de bras mobiles (20) disposés à proximité ou au niveau de la seconde extrémité (6) ;
dans lequel l'actionneur (16) et les un ou plusieurs éléments de bras mobiles (20) sont raccordés de manière fonctionnelle de telle sorte que l'actionnement de l'actionneur (16) amène les un ou plusieurs éléments de bras (20) à se déplacer entre une configuration rétractée et une configuration déployée, dans lequel, dans la configuration déployée, au moins une partie des un ou plusieurs éléments de bras (20) est disposée à l'extérieur du corps (2),
dans lequel, dans la configuration rétractée, aucune partie des un ou plusieurs éléments de bras mobiles (20) ne fait saillie au-delà d'un périmètre extérieur du corps (2), **caractérisé en ce que**
les un ou plusieurs éléments de bras (20) s'étendent entre une extrémité de raccordement (22) et une extrémité libre (24), et le corps (2) comprend un ou plusieurs éléments d'ouvertures (26) disposés à proximité ou au niveau de la seconde extrémité (6), et les un ou plusieurs éléments d'ouvertures (26) sont configurés de telle sorte qu'au moins l'extrémité libre (24) d'un ou plusieurs éléments de bras (20) peut se déplacer depuis une région à l'intérieur du corps (2) et à travers l'ouverture (26) vers une région à l'extérieur du corps (2), et vice versa.

2. Dispositif (1) selon la revendication 1, dans lequel l'actionneur (16) est agencé pour être mobile à l'intérieur du corps (2).

3. Dispositif (1) selon la revendication 1 ou la revendication 2, dans lequel l'actionneur (16) comprend une partie mobile agencée pour être reçue de manière mobile au moins partiellement à l'intérieur du corps (2).

4. Dispositif (1) selon la revendication 3, dans lequel :
la partie mobile est raccordée de manière fonctionnelle aux un ou plusieurs éléments de bras (20) ; et/ou
dans lequel la partie mobile est actionnable pour se déplacer dans la direction longitudinale par rapport au corps (2) ; et/ou
dans lequel la partie mobile est actionnable pour tourner autour de l'axe longitudinal par rapport au corps (2) ; et/ou
dans lequel la partie mobile est agencée pour être mobile manuellement par un utilisateur ou dans lequel l'actionneur (16) comprend un actionneur mécanique, électronique ou électromécanique, éventuellement dans lequel l'actionneur (16) comprend un solénoïde, et en outre éventuellement dans lequel la partie mobile comprend une armature mobile du solénoïde.

5. Dispositif (1) selon l'une quelconque des revendications précédentes, comprenant une partie d'actionnement (18) disposée à proximité ou au niveau de la première extrémité (4), la partie d'actionnement (18) étant configurée pour permettre ou aider un utilisateur à faire fonctionner ou actionner l'actionneur (16), éventuellement dans lequel la partie d'actionnement (18) comprend une poignée, un levier, un bouton, un écran tactile, un capteur tactile, ou un commutateur.

6. Dispositif (1) selon la revendication 5, dans lequel la partie d'actionnement (18) est agencée de telle sorte que le mouvement de la partie d'actionnement (18) provoque le mouvement de l'actionneur (16).

7. Dispositif (1) selon l'une quelconque des revendications précédentes, dans lequel chacun des un ou plusieurs éléments de bras (20) comprend un élément supérieur (20i') et un élément inférieur (20i") raccordé de manière pivotante à l'élément supérieur (20i'), dans lequel un élément parmi l'élément supérieur (20i') et l'élément inférieur (20i") est directement couplé à l'actionneur (16) et l'autre élément parmi l'élément supérieur (20i') et l'élément inférieur (20i") est directement couplé au corps (2), éventuellement dans lequel l'un ou l'autre ou les deux de l'élément inférieur (20i'') et de l'élément supérieur (20i') sont couplés de manière pivotante au corps (2) et à l'actionneur (16), éventuellement dans lequel les un ou plusieurs éléments d'ouvertures (26) sont configurés de telle sorte qu'au moins une partie d'un ou plusieurs éléments de bras (20) s'étend à travers ceux-ci.

8. Dispositif (1) selon l'une quelconque des revendications précédentes, dans lequel l'extrémité de raccordement (22) d'un ou plusieurs éléments de bras (20) est raccordée de manière fonctionnelle à l'actionneur (16).

9. Dispositif (1) selon l'une quelconque des revendications précédentes, dans lequel les ouvertures (26) sont espacées du point terminal de la seconde extrémité (6) du corps (2) dans la direction longitudinale.

10. Dispositif (1) selon l'une quelconque des revendications précédentes, dans lequel, dans la configuration déployée, au moins une partie des un ou plusieurs bras (20) est orientée sensiblement perpendiculairement à un axe longitudinal du corps (20).

11. Dispositif (1) selon la revendication 3 ou l'une quelconque des revendications 4 à 11 lorsqu'elle dépend de la revendication 3, comprenant un moyen de fixation temporaire actionnable pour empêcher temporairement l'actionneur (16) de se déplacer par rapport au corps (2).

12. Dispositif (1) selon l'une quelconque des revendications précédentes, comprenant au moins une source de lumière, éventuellement dans lequel la source de lumière comprend un ou plusieurs éléments de LED (28) et/ou éventuellement dans lequel la source de lumière est disposée à proximité ou au niveau de la seconde extrémité (6) du corps (2).

13. Dispositif (1) selon l'une quelconque des revendications précédentes, dans lequel le dispositif (1) est configuré pour être utilisé avec une source de lumière externe (221).

14. Dispositif (1) selon la revendication 12 ou la revendication 13, comprenant un ou plusieurs éléments de guides de lumière configurés pour diriger la lumière depuis la source de lumière vers une partie souhaitée du dispositif (1).

15. Dispositif (1) selon l'une quelconque des revendications précédentes :
comprenant un canal fluidique approprié pour acheminer un courant de fluide à travers celui-ci, éventuellement dans lequel le canal fluidique comprend une entrée de fluide (32) disposée à proximité ou au niveau de la première extrémité (4) du corps (2) et une sortie de fluide disposée à proximité ou au niveau de la seconde extrémité (6) du corps (2) ; et/ou
le dispositif (1) incluant un élément d'étanchéité (211) configuré pour fournir un joint, lors de l'utilisation, autour d'au moins une partie d'un périmètre du corps (2).
